# EUROPEAN PATENT APPLICATION

(11) **EP 1 829 564 A1**
(43) Date of publication of application: **05.09.2007**
(21) Application number: 06075368.8
(22) Date of filing: 17.02.2006
(51) Int. Cl.: A61L 24/00

(54) **Osteoinductive calcium phosphate**

(71) Applicant: Progentix B.V. i.o., 3723 MB Bilthoven (NL)
(72) Inventor: De Bruijn, Joost Dick, 3817 JL Amersfoort (NL); Yuan, Huipin, 3706 AA Zeist (NL)
(74) Representative: Winckels, Johannes Hubertus F.

(57) **Abstract**

The invention relates to an osteoinductive biomaterial based on calcium phosphate, wherein the material is in the form of microparticles having a particle size ranging from about 50 to about 1500 µm, and wherein the porosity of the material consists essentially of micropores in a size range of 0.1-3 µm.

## Description

### FIELD OF THE INVENTION

The invention relates to an osteoinductive material, to a method for preparing said material and to a material produced that way.

### BACKGROUND OF THE INVENTION

Autologous bone harvested from the patient's own bone is the gold standard bone substitute for repairing large bone defects. However, the amount of autologous bone harvestable from a patient is limited and the bone subtraction itself poses significant health risks and results in loss of structural integrity of the remaining bone.

Developments in tissue engineering have provided synthetic implants, for instance in the form of scaffold materials, which allow attachment of bone cells and ingrowth of new bone tissue and subsequent deposition of new bone mineral. The synthetic materials may either be grafted ex vivo with bone cells prior to implantation or may be implanted as naked scaffolds that attract bone cells from the periphery to the site of the implant.

Recent advances in tissue engineering have produced a variety of valuable scaffold materials. Calcium phosphates such as hydroxyapatite (HA; the mineral phase of bone), bicalcium phosphate (BCP) and α- or β-tricalcium phosphate (TCP) are known to possess both osteoconductive (bioactive) as well as osteoinductive properties and provide very suitable scaffold materials. The bioactive nature of calcium phosphates allows them to function as a template for new bone formation by osteogenic cells through deposition of new mineral material at the scaffold's surface and is an important feature of the scaffold material. The osteoinductive nature of calcium phosphates is a qualitative feature, i.e. the capacity to induce the development of the new bone tissue, thereby enhancing the rate of deposition of new mineral depends on various material parameters. Bone induction is generally defined as the mechanism by which a mesenchymal tissue is induced to change its cellular structure to become osteogenic.

In general, porous calcium phosphates have been found to exhibit osteoinductivity. For instance, Yamasaki et al., in Biomaterials 13:308-312 (1992), describe the occurrence of heterotopic ossification (formation of new bone in tissue that do not normally ossify) around porous hydroxyapatite ceramic granules, but not around dense granules. The porous granules range in size from 200 to 600 µm, and have a continuous and interconnected microporosity of which the pores range in diameter from 2 to 10 µm.

US Pat No. 6511510 describes a biocompatible and biodegradable calcium phosphate that exhibits improved osteoinductivity over the porous hydroxyapatite granules of Yamasaki *et al*. The biodegradable calcium phosphate has a total porosity of 20 to 90%, and encompasses both macropores ranging in size from 0.1 to 1.5 mm, as well as micropores ranging in size from 0.05 to 20 µm. The biodegradable calcium phosphate material is produced by mould casting and blocks can subsequently be granulated or cut to smaller size particles. The material, when implanted, is suitable to function as a (temporary) substitute for bone.

Despite the availability of the above materials, it would be advantageous if bomaterials could be provided with even better osteoinductive properties, i.e. that result in even faster and more profound bone formation. It would also be advantageous if such osteoinductive materials could be easily introduced in the body of the mammal, most preferably such that they provide an easily implantable and effective scaffold material for the production of new bone in both osseous and non-osseous sites. Such material would be of much use for the production of *de novo* autologous bone, which might subsequently be used as bone substitute for repairing large bone defects.

### SUMMARY OF THE INVENTION

The present invention provides a calcium phosphate material having excellent osteoinductive properties. It has surprisingly been found that a material in the form of porous microparticles, wherein the particles have a particle size ranging from about 100 to about 500 µm, and wherein the porosity of the material is based primarily on the presence of micropores in a size range of 0.1-3 µm, preferably 0.5-2 µm, has osteoinductive properties that are improved over the materials of the prior art. Hence, the present invention in a first aspect provides an osteoinductive biomaterial based on calcium phosphate, wherein the material is in the form of microparticles having a particle size ranging from about 50 to about 1500 µm, preferably 100-500 µm, and wherein the porosity of the material consists essentially of micropores in a size range of 0.1-3 µm, preferably 0.5-2 µm, most preferably 0.5-1.5 µm. Thus, it is a feature of the material that it exhibits microporosity (pores <5 µm), preferably interconnected microporosity, and that it is essentially free of macropores (pores ranging in size from 0.1 to 1.5 mm).

In a preferred embodiment, the biomaterial of the invention is composed of crystals of the calcium phosphate material having a size of between 0.05 and 3 µm, preferably between 0.5 and 2 µm

The biomaterial of the invention is preferably in the form of microparticles having a particle size ranging from about 200 to about 300 µm, more preferably 212-300 µm.

The material of the invention shows excellent osteoinductive behaviour in living tissue. The formation of bone tissue at the surface of the material of the invention assists in a favourable acceptation of an implant made of said material. Moreover, the formation of the bone tissue accelerates the recovery of any damage in the bone structure, which forms the reason for applying the implant.

An advantage of the material of the present invention is that it has excellent flowing properties. The sand-like constitution of the material allows it to be injected without an additional fluidic carrier. Thus, the material itself may be used as an injectable, although it may also be used in admixture with for instance a liquid carrier.

In a preferred embodiment, the calcium phosphate on which the biomaterial of the invention is based is chosen from the group consisting of octacalcium phosphate, apatites, such as hydroxyapatite and carbonate apatite, whitlockites, such as β-tricalcium phosphate and α-tricalcium phosphate, and combinations thereof. More preferably the calcium phosphate is resorbable biphasic calcium phosphate (BCP) and resorbable tricalcium phosphate, most preferably β- tricalcium phosphate.

In another aspect, the present invention relates to the biomaterial for use as a medical implant material or tissue scaffold.

In yet another aspect, the invention provides the use of the osteoinductive biomaterial for inducing the formation of bone tissue in a living organism. The material may be used as an implant material for the production of autologous bone in both osseous sites and non-osseous sites, both in orthopedic as well as in dental and veterinary applications, or for the production of a medical implant or device.

In yet another aspect, the invention provides a method for producing an osteoinductive biomaterial based on calcium phosphate, comprising providing an aqueous slurry of a calcium phosphate powder, a foaming agent and optionally a porogenic agent in water; subjecting the slurry to conditions which cause foaming of said slurry; drying the resultant foamed slurry and optionally removing the porogenic agent, and sintering the dried and foamed slurry to obtain a porous sintered calcium phosphate ceramic; milling the sintered calcium phosphate ceramic to particles and collecting the particles having a particle size ranging from about 50 to about 1500 µm, preferably 100-500 µm.

In a preferred embodiment of such a method, the particles may be collected by using 212 and 300 µm sieves, which results in the collection of microparticles ranging in size from 212-300 µm.

Preferably the calcium phosphate powder used in methods of the invention is composed of crystals having (an average) crystal size between 0.05 and 3 µm, more preferably between 0.5 and 2 µm. The calcium phosphate powder is preferably TCP or BCP powder.

The foaming agent used in the method is preferably hydrogen peroxide and the optional porogenic agent preferably comprises of naphthalene particles, which may be removed from the "green body" (i.e. from the foamed and dried slurry, prior to sintering) by evaporation at 80-100°C. Preferably the "green body" is a porous "green body" produced by foaming of said slurry.

In another preferred embodiment, the conditions which cause foaming of said slurry comprise heating of the slurry to about 50-70°C.

Preferably the sintering of the dried and foamed slurry is performed at a temperature of between 800 and 1300°C.

In still another preferred embodiment the collected microparticles are subsequently cleaned ultrasonically with acetone, ethanol and/or water, and optionally dried and sterilized.

In another aspect, the present invention provides an osteoinductive biomaterial obtainable by a method of the invention.

### BRIEF DESCRIPTION OF THE DRAWINGS

Figures 1-3 show the physicochemical properties of an osteoinductive granular tricalcium phosphate ceramic material according to the invention as described in more detail in the Example.
Figure 1 show the XRD pattern of the material.
Figure 2 is a morphological view of micropores in material (SEM image).
Figure 3 presents a plot of incremental pore volume versus average micropore diameter as determined by mercury intrusion (indicating that incremental pore volume peaks at pore size of 1.1 µm).
Figure 4 shows the two implant materials used in the Example: microporous TCP particles having particle size 1-3mm (panel A: comparative), and 212-300 µm (panel B: according to the present invention).
Figure 5 represents micrographs of histological preparations of retrieved implants as described in the Example. Bone formation (pink coloration) after 12-week implantation in muscle of dogs associated with an implant based on microporous particles of TCP having a particle size of 1-3 mm (left hand side) compared to the bone formation associated with an implant based on microporous particles of TCP having a particle size of 212-300 µm (right hand side)

### DETAILED DESCRIPTION OF THE INVENTION

A "biomaterial" as defined herein is a synthetic material used to replace part of a living system or to function in intimate contact with living tissue.

A biomaterial in aspects of the present invention may be based on any calcium phosphate (CaP), such as a CaP obtained by precipitation from an aqueous solution at low temperature or by a high temperature (thermal) process. Highly preferred calcium phosphates are the calcium orthophosphates. The term "calcium orthophosphate" as used herein refers to a family of compounds, each of which contains a calcium cation, Ca²⁺, and a phosphate anion, PO₄³⁻. Under this definition, there are multiple calcium orthophosphates, including monocalcium orthophosphate (monobasic), dicalcium orthophosphate (dibasic), tricalcium orthophosphate (tribasic), and hydroxyapatite (penta calcium triphosphate).

Although this invention is described mainly in terms of calcium orthophosphate, other suitable materials useful herein include for instance calcium pyrophosphates (e.g., dicalcium diphosphate (Ca₂P₂O₇, synonym: calcium pyrophosphate), calcium pyrophosphate dihydrate (CPPD, Ca₂P₂O_{7.}2H₂O), and calcium dihydrogen diphosphate (CaH₂P₂O₇; synonyms:
acid calcium pyrophosphate, monocalcium dihydrogen pyrophosphate)), and polyphosphate ((CaP₂O₆)ₙ, n≥ 2; synonyms: calcium metaphosphates, calcium polymetaphosphates), and combinations of the various phosphates.

Non-limiting examples of the calcium phosphate compound that may be used in aspects of the invention are:
- α-tricalcium phosphate (α-TCP, α-Ca₃(PO₄)₂, synonyms: whitlockite, tricalcium phosphate, calcium phosphate tribasic), either anhydrous or as hydrate;
- β-tricalcium phosphate (β-TCP, β-Ca₃(PO₄)₂, synonyms: whitlockite, tricalcium phosphate, calcium phosphate tribasic), either anhydrous or as hydrate;
- amorphous calcium phosphate (ACP, Ca₃(PO₄)₂.nH₂O, n = 3 - 4.5, Ca/P ratio = 1.5)
- apatite (calcium fluoro-phosphate, Ca₅(F,Cl,OH)(PO₄)₃)
- calcium dihydrogen phosphate (Ca(H₂PO₄)₂);
- calcium dihydrogen phosphate hydrate (Ca(H₂PO₄)₂.H₂O)
- calcium hydrogen phosphate hydrate (CaHPO₄.2H₂O);
- calcium hydrogen phosphate, anhydrous (CaHPO₄),
- calcium-deficient hydroxyapatite or precipitated hydroxyapatite (PHA) Ca₁₀₋ₓ(HPO₄)ₓ(PO₄)₆₋ₓ(OH)₂₋ₓ (0 ≤ x ≤ 1) with Ca/P ratio varying from 1.5 to 1.67
- carbonate apatite (Ca₅(PO₄,CO₃)₃F)
- dicalcium phosphate anhydrous (DCPA, CaHPO₄)
- dicalcium phosphate dihydrate (DCPD, CaHPO₄.2H₂O);
- fluoroapatite (FA, Ca₅(PO₄)₃F);
- hydroxyapatite (HA, Ca₅(PO₄)₃OH, synonyms: (penta)calcium triphosphate);
- monocalcium phosphate anhydrous (MCPA, Ca(H₂PO₄)₂);
- monocalcium phosphate monohydrate (MCPM, Ca(H₂PO₄)₂.H₂O);
- octacalcium phosphate (OCP, Ca₈H₂(PO₄)6.5H₂O);
- oxyapatite (Ca₁₀(PO₄)₆O);
- tetracalcium phosphate (TTCP, Ca₄(PO₄)₂O);
- mixtures of two or more of the above such as mixtures of MCPM or MCPA with another CaP such as α-tricalcium phosphate or β-tricalcium phosphate, and
- composites of two or more of the above such as composites of β-TCP and hydroxyapatite (Ca/P - 1.67), e.g. biphasic calcium phosphate (BCP).

The calcium phosphates, particularly in case they are derived from natural sources, may be calcined prior to use. As the osteoinductive biomaterial of the invention is preferably used as an implant in living tissue, the calcium phosphate is preferably synthetic. Moreover, the osteoinductive biomaterial is preferably both sufficiently biocompatible and sufficiently biodegradable for use as an implant in living tissue. Thus, the calcium phosphate on which the osteoinductive biomaterial is based is preferably (bio)resorbable, meaning that it exhibits chemical dissolution and cell-mediated resorption when placed in a mammalian body.

An osteoinductive biomaterial according to the invention is preferably based on HA, α-TCP, β-TCP, octacalcium phosphate, or combinations thereof, such as BCP. An osteoinductive biomaterial according to the invention is most preferably based on a BCP or TCP.

The material of the present invention is porous. The porosity of the material consists essentially of micropores in a size range of 0.1-3.0 µm, preferably of 0.5-2 µm, more preferably of 0.5 -1.5 µm. The total porosity ranges from 20 to 90%, preferably from 40 to 70%.

The invention further relates to a process for preparing an osteoinductive biomaterial as described above and to an osteoinductive biomaterial obtainable by said method.

A method of the invention for producing an osteoinductive biomaterial based on calcium phosphate, comprises the steps of providing an aqueous slurry of a calcium phosphate powder, a foaming agent and optionally a porogenic agent in water; subjecting the slurry to conditions which cause foaming of said slurry; drying the resultant foamed slurry and optionally removing the porogenic agent, and sintering the dried and foamed slurry to obtain a porous sintered calcium phosphate ceramic; milling the sintered calcium phosphate ceramic to particles and collecting the particles having a particle size ranging from about 100 to about 500 µm.

The concentration of the foaming agents (i.e. H₂O₂) is range from 0.1% to 10.0%, the ratio of foaming agents (H₂O₂, 0.1-10.0%) for 100 powder is between 10 and 300ml, and the ratio of porogenic agent (i.e. naphthalene particles, <1400um) to 100g powder is between 0-150 g.

In order to prepare the present osteoinductive biomaterial, a calcium phosphate based material is sintered under such conditions, that an osteoinductive biomaterial as described above is obtained.

Preferably the calcium phosphates are formed by a process involving sintering, in which high temperatures (800 -1300°C), pressure, and one or more calcium phosphates are being used to form the final product. The properties of the final product can be adjusted by selecting specific combinations of temperature, pressure and calcium phosphate starting materials. For example, pure HA may be formed by using an apatite with a Ca/P ratio of 1.67, whereas TCP may be formed by using an apatite with a Ca/P ratio of 1.5. When for instance apatites with varying Ca/P ratios are sintered, different amounts of HA and TCP are formed in the final ceramic, resulting in biphasic calcium phosphates (BCPs). Another factor that is determined by the sintering parameters is the residual microporosity. The microporosity of the ceramics may in some embodiments of a method for their production be due to gaps left between the sintered particles, which is - in turn - influenced by the crystallization of the CaP used.

In accordance with a preferred embodiment of the invention, the biomaterial is made up of crystals. Preferably, the size of the crystals is similar to the size of the micropores. Thus, the size of the crystals lies preferably between 0.1 and 3 µm, more preferably between 0.5 and 2 µm, still more preferably between 0.5 and 1.5 µm.

Dense and porous calcium phosphates ceramics are generally produced by different sintering techniques. Dense ceramics are produced by compaction under high pressure, resulting in a frequently called "green" state, and are sintered after the compaction process. Porous calcium phosphates of the invention may for instance be produced by using appropriate-sized naphthalene particles as porogenic agent, incorporated in the aqueous slurry of the calcium phosphate starting material. After compaction under high pressure, removal of naphthalene is accomplished by sublimation, which leaves a porous green state. The integrity of this porous green state is maintained through the sintering step. The use of naphthalene particles is for instance described in Moore et al. (2001) Australian and New Zealand Journal of Surgery 71:354- 361 and Li et al. (2003) Journal of the American Ceramic Society 86:65-72.

Another method of producing porous ceramics may for instance rely on the decomposition of hydrogen peroxide to generate a pore-filled structure. In such cases, the hydrogen peroxide functions as a foaming agent, whereby the escaping gas produces the desired interconnectivity of the pores. The skilled person will understand that also other foaming agents may be used to produce a porous green body, which, upon drying, may be sintered to produce a calcium phosphate based material having the required porosity. The concentrations of the calcium phosphate in the slurry is preferably such that no additional stabilisers or thickeners are required.

As explained above, the size of the pores may be controlled by the size of the calcium phosphate crystals of the starting material, by the type and amount of foaming agent, by the conditions to which a slurry of the starting material is subjected for obtaining the foamed "green body", and by the type, particle size and amount of the optional porogenic agent. Preferably, the sintering is carried out at a temperature between 800 °C and 1250 °C. The duration of the sintering step may suitably be chosen between 1 and 10 hours, preferably between 7 and 9 hours.

Upon sintering, the material is optionally treated with an aqueous solution of an organic acid and washed. The washing may suitably be performed using ethanol, water or a combination thereof.

An important aspect of the invention is the physical structure of the osteoinductive biomaterial. The material is in the form of microparticles or granules, i.e. it is a granular, loose material consisting of particles in a size range of 50-1500 µm, preferably 100-500 µm, more preferably 200-300 µm, and most preferably 212-300 µm in particle size. Therefore, after the sintering and optional washing, the material is ground, for instance in a ball mill, to produce a relatively coarse powder that comprises microparticles in a size range of 50-1500 µm, preferably 100-500 µm, more preferably 200-300 µm, and most preferably 212-300 µm in particle size. Specific size ranges may be retrieved by using sieves, for instance 212 and 300 µm sieves.

Finally, it is preferred to subject the obtained microparticles of the osteoinductive biomaterial to a sterilisation treatment, such as a steam, ethylenoxid or gamma sterilisation.

The granular biomaterial may be used in the form of an injectable, either alone in the form of a powder or in combination with a liquid carrier in the form of a paste.

The biomaterial of the present invention may for instance be used as a calcium phosphate cement or it may be used for inducing the formation of bone tissue in a living organism.

The biomaterial of the present invention may suitably be used as an implant material, i.e. as a scaffold, for the production of autologous bone in a non-osseous site. This ability is due to the highly osteoinductive properties of the material.

The granular biomaterial may thus be used as a medical implant or medical device formed of a calcium phosphate. It is also possible that the biomaterial is used in combination with a medical implant of a different material, such as a metal or a polymeric material, on which the osteoinductive biomaterial according to the invention is present in the form of a coating.

It should be noted that the various uses of the material of the present invention include general surgical applications in bone repair, as well as applications in dental surgery.

The invention will now be illustrated by way of the following. non-limiting example.

### EXAMPLE

### 1.1. Preparation of the materials

### Tricalcium phosphate ceramic.

TCP powder (Plasma Biotal, UK) was mixed with H₂O₂ solution (1.0-2.0% in water, 100-200 ml/100 g TCP powder) and naphthalene particles (500-1400 µm, 0-150 g/100 g powder), and foamed at 50-70 °C to get porous green bodies. After dried and naphthalene was evaporated at 80-100 °C, the green bodies were sintered at 1100 °C for 8 hours. Ceramic particles (1.0-3.0 mm) and microparticles (212-300 µm) were made and cleaned ultrasonically with acetone, ethanol and water, and finally dried at 80°C.

### 1.2. Characterization of the materials

The chemistry of the material was analyzed with XRD and FTIR, micropores were analyzed with SEM (morphology) and Mercury intrusion (micropore size).

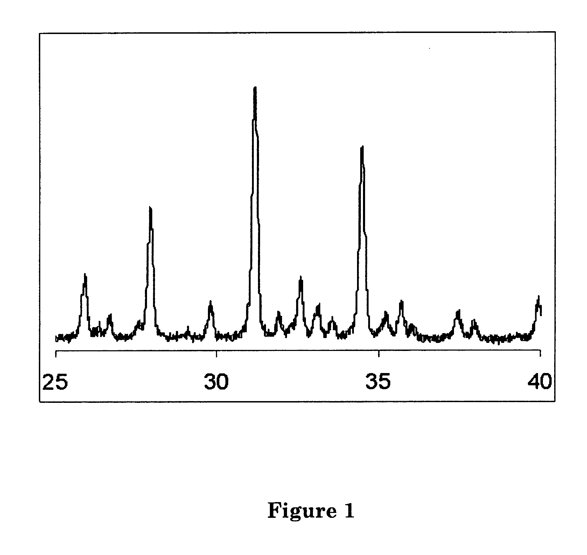

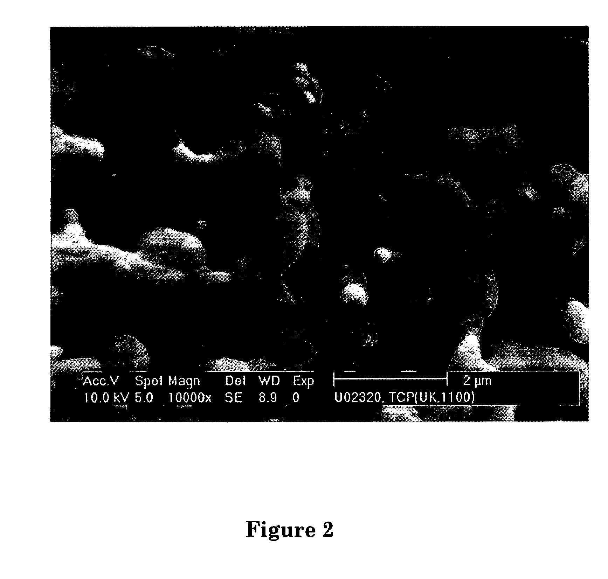

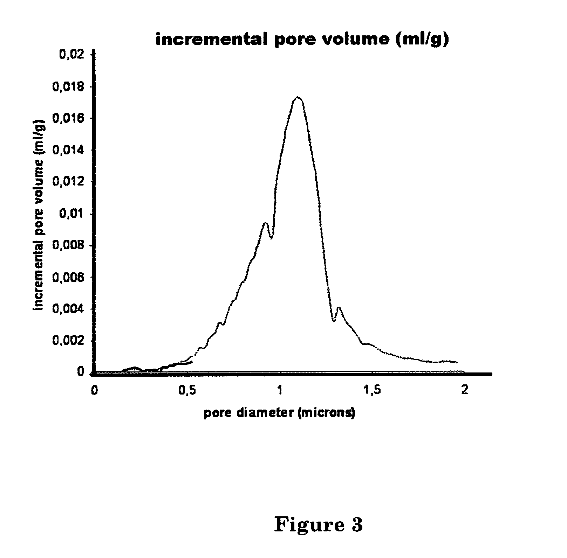

The results are presented in Figures 1-3, and show that the material prepared is chemically β-tricalcium phosphate containing a trace of Hydroxyapatite (Figure 1). Interconnected micropores less than 2 µm distribute homogeneously in the material (Figure 2). The size of the micropore size is between 0.5-1.5 µm as measured with mercury intrusion (Figure 3).

### 1.3 Animal study and histology

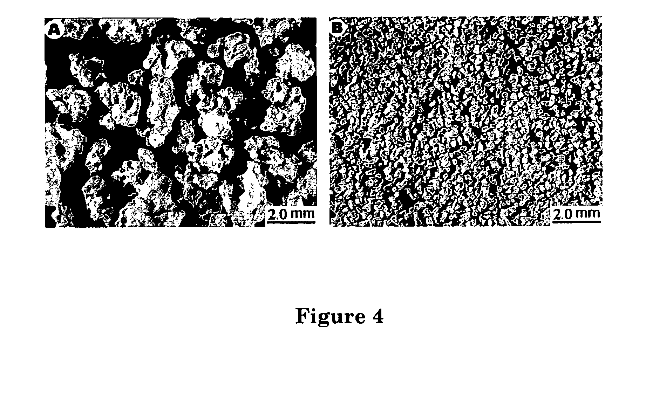

Implants consisted of a volume of 1.0 cc of ceramic particles. A control implant consisted of an implant with particle size of 1-3 mm (comparative example; Figure 4A) and the test-implant consisted of an implant with particle size of 212-300 µm (material according to the present invention; Figure 4B). Both types of implants were implanted in back muscles of dog. Eight dogs received both implants for 12 weeks. After 12 weeks, the implants were retrieved, including some surrounding tissues and were fixed in 10% buffered formation (pH=7.4). The fixed samples were dehydrated with series ethanol solutions (70%, 80%, 90%, 96% and 100% x 2) and finally embedded in MMA. Non-decalcified sections (10-20 µm) were made and stained with methylene blue and basic fuchsin for histological observation and histomorphometrical analysis regarding formation. Histomorphometry was performed on the sections across the middle of the implants with regard to the percentage of the formed bone in the available space.

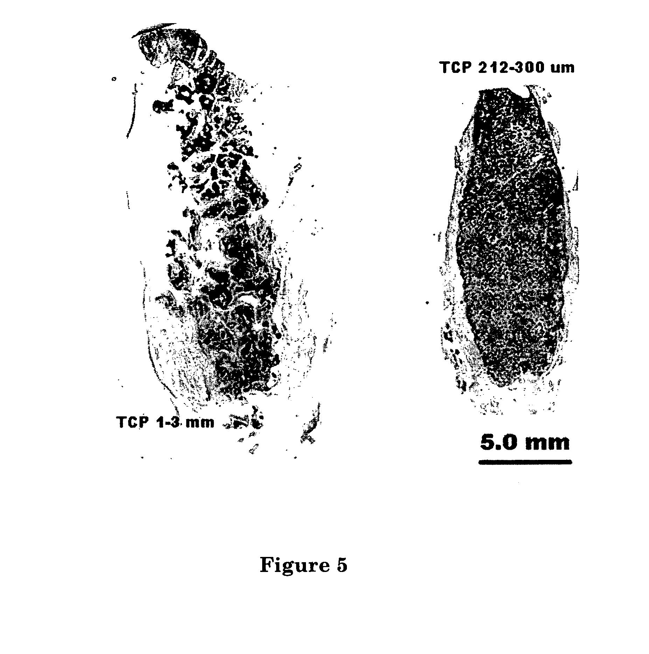

The size or volume of the implants decreased (less than 1cc) after intramuscular implantation in dogs for 12 weeks, indicating the resorbable nature of the TCP. Moreover, the remaining size of the implant of 1-3 mm particles is bigger than implant based on the particles with a size of 212-300 µm, indicating that the 212-300 µm microparticles are resorbed faster than 1-3 mm particles. Resorption of the materials was also observed histologically (Figure 5). Intact TCP particles were visible in TCP implants based on the 1-3 mm particles, while most TCP microparticles (212-300 µm) broke down and resorbed.

Bone formation was seen in 6 out of 8 TCP implants of 212-300 µm microparticles and 8 out of 8 TCP implants of 1-3mm. The most dramatic effect, however, was observed for the implant based on the 212-300 µm microparticles. Whereas bone formation associated with the particles of 1-3 mm was limited and confined to the particles themselves (Figure 5, left hand side), massive and widespread bone formation was found to be associated with the microparticles of 212-300 µm and the bone was found to have formed primarily between the microparticles (figure 5, right hand side). Moreover, most of the microparticles were resorbed after 12-week implantation and the implants were actually transformed into a "real" autologous bone.

### 1.4 Discussion and conclusion

The invention presents an enhanced inductive bone formation associated with implants having microporous calcium phosphates having micropores in size of 0.5-1.5 µm. Enhanced bone formation is observed by using this material in a particulate form and using specific particle size in the implants (i.e. microparticles, for instance 212-300 µm).

This approach now demonstrates to possibility of producing a real autologous bone in a non-osseous site and complete resorption of the calcium phosphate scaffold material.

## Claims

1. An osteoinductive biomaterial based on calcium phosphate, wherein the material is in the form of microparticles having a particle size ranging from about 50 to about 1500 µm, and wherein the porosity of the material consists essentially of micropores in a size range of 0.1-3 µm.

2. Biomaterial according to claim 1, wherein the porosity of the material consists essentially of micropores in a size range of 0.5-2 µm.

3. Biomaterial according to claim 1 or 2, wherein the microparticles have a particle size ranging from about 200 to about 300 µm, preferably 212-300 µm.

4. Biomaterial according to any of the preceding claims, which is composed of crystals having a crystal size between 0.05 and 3 µm, preferably between 0.5 and 2 µm.

5. Biomaterial according to any of the preceding claims, wherein the calcium phosphate is chosen from the group consisting of octacalcium phosphate, apatites, such as hydroxyapatite and carbonate apatite, whitlockites, such as β-tricalcium phosphate and α-tricalcium phosphate, and combinations thereof.

6. Biomaterial according to any of the preceding claims, wherein the calcium phosphate is resorbable biphasic calcium phosphate (BCP) or resorbable tricalcium phosphate, preferably β- tricalcium phosphate.

7. Biomaterial according to any of the preceding claims for use as a medical implant material or tissue scaffold.

8. Use of a biomaterial as defined in any one of claims 1-7 for inducing the formation of bone tissue in a living organism.

9. Use of a biomaterial as defined in any of claims 1-7 as an implant material alone or combined with growth factors or/and cells for the production of autologous bone in a non-osseous site.

10. Use of a biomaterial as defined in any of claims 1-7 for the production of a medical implant or device alone or combined with growth factors or/and cells.

11. Use according to any one of claims 8-10 in dental surgery.

12. A method for producing an osteoinductive biomaterial based on calcium phosphate, comprising providing an aqueous slurry of a calcium phosphate powder, a foaming agent and optionally a porogenic agent in water; subjecting the slurry to conditions which cause foaming of said slurry; drying the resultant foamed slurry and optionally removing the porogenic agent, and sintering the dried and foamed slurry to obtain a porous sintered calcium phosphate ceramic; milling the sintered calcium phosphate ceramic to particles and collecting the particles having a particle size ranging from about 50 to about 1500 µm.

13. Method according to claim 12, wherein the particles are collected by using 212 and 300 µm sieves.

14. Method according to claim 12 or 13, wherein the calcium phosphate powder is composed of crystals having a crystal size between 0.05 and 3 µm, preferably between 0.5 and 2 µm.

15. Method according to any one of claims 12-14, wherein the foaming agent is hydrogen peroxide.

16. Method according to any one of claims 12-15, wherein the porogenic agent comprises of naphthalene particles, and wherein the porogenic agent is removed by evaporation at 80-100°C.

17. Method according to any one of claims 12-16, wherein said conditions which cause foaming of said slurry comprise heating of the slurry to about 50-70°C.

18. Method according to any one of claims 12-17, wherein foaming of said slurry produces porous green bodies.

19. Method according to any one of claims 12-18, wherein the dried and foamed slurry is sintered at a temperature of between 800 and 1300°C.

20. Method according to any one of claims 12-19, wherein said calcium phosphate powder is TCP or BCP powder.

21. Method according to any one of claims 12-20, wherein said collected microparticles are subsequently cleaned ultrasonically with acetone, ethanol and/or water, and optionally dried and sterilized.

22. An osteoinductive biomaterial obtainable by a method according to any one of claims 12-21.
